# EUROPEAN PATENT SPECIFICATION

(11) **EP 1 578 433 B1**
(45) Date of publication and mention of the grant of the patent: **18.08.2010**
(21) Application number: 03797114.0
(22) Date of filing: 17.09.2003
(51) Int. Cl.: A61K 36/67, A61P 17/00

(54) **USE OF POTHOMORPHE UMBELLATA EXTRACT AND GEL COMPOSITION ON BASIS OF POTHOMORPHE UMBELLATA EXTRACT**
VERWENDUNG EINES POTHOMORPHE UMBELLATA-EXTRAKTS UND AUF POTHOMORPHE UMBELLATA EXTRAKT BASIERENDE GELZUSAMMENSETZUNG.
UTILISATION D'EXTRAIT DE POTHOMORPHE UMBELLATA ET GEL COMPOSITION A BASE D'EXTRAIT DE POTHOMORPHE UMBELLATA

(30) Priority: 18.09.2002 BR 0204130
(43) Date of publication of application: 28.09.2005
(73) Proprietor: Fundacao de Amparo a Pesquisa do Estado de Sao Paulo - FAPESP, CEP-05468-150 Sao Paulo (BR); Universidade De São Paulo-USP, Butanta, CEP 05508-900 Sao Paulo (BR)
(72) Inventor: BARROS, Silvia, Berlanga, de, Moraes, CEP 05018-001 Sao Paulo (BR); ROPKE, Cristina, Dislich, CEP 02341-010 Sao Paulo (BR)
(74) Representative: Wenger, Joel-Théophile
(86) International application number: PCT/BR2003/000134
(87) International publication number: WO 2004/026323

(56) References cited:
- DE-A1- 19 933 857
- PATENT ABSTRACTS OF JAPAN & JP 2001 122763 A (LION CORP) 08 May 2001
- ISOBE T. ET AL.: 'Antibacterial constituents against helicobacter pylori of Brazilian medical plant, Pariparoba, Yakugaku zasshi' JOURNAL OF PHARMACEUTICAL SOCIETY OF JAPAN vol. 122, no. 4, April 2002, pages 291 - 294, XP001223546 & DATABASE MEDLINE [Online] Database accession no. (NLM11968842)
- FELZENSZWALB I. ET AL.: 'Absence of mutagenicity of potomorphe umbellata and potomorphe peltata in the salonella/mammlian- microsome mutagenicity assay' BRAZILIAN JOURNAL OF MEDICAL AND BIOLOGICAL RESEARCH vol. 20, no. 3-4, 1987, pages 403 - 405, XP001223569 & DATABASE MEDLINE [Online] Database accession no. (NLM3330461)
- DATABASE WPI Week 199742, Derwent Publications Ltd., London, GB; AN 1997-453955, XP002992582 & JP 9 208 483 A (KAO CORP) 12 August 1997

## Description

### Fields Encompassed by Invention

The present invention is applied to Pharmacy and Medicine fields and relates to the use of the *Pothomorphe umbellata* extract in order to prepare dermocosmetic or pharmaceutical compositions for the treatment and/or prevention of photodamage in skin, cutaneous aging and/or skin cancer. Another form of usage is the application of dermocosmetic or pharmaceutical compositions on basis of *Pothomorphe umbellata* extract, in order to treat and/or prevent the damage caused to the skin by the excessive exposure to ultraviolet rays of the sun and to artificial tanning lamps and alterations caused by chronological aging.

### Background Art

The skin is the human body largest organ and it is constantly exposed to the free radicals generating sources, as ultraviolet radiation, air pollutants and ionizing radiation. The multiple exposure to solar radiation, without appropriate protection, can produce undesirable effects, such as photoaging and photocarcinogenesis (TEDESCO, *et al*., 1997).

The aging is followed by a progressive reduction of the organic functions and an increase in the vulnerability to diseases. Particularly concerning the skin, many functional decreases and pathologies connected to peoples age are the cumulative result of environmental insults suffered during intrinsic aging (GILCHREST & YAAR, 1992). Intrinsic aging is defined as the changes that occur in all the individuals throughout time, while photoaging is the overlap of changes attributed to solar exposure throughout the aging process (GILCHREST & YAAR, 1998). Unlike intrinsic aging, not only is the photoaged skin characterized by an exacerbation of the changes caused by chronological aging, but also for the presence of qualitatively different alterations induced by the exposure to sun(GILCHREST, 1996).

The consequent degenerative alterations of repetitive exposures to solar radiation occur firstly areas which suffer more exposure, such as face, neck, arms and hands. (DELLA CARBONARE & PATHAK, 1992). Macroscopically, the photoaged skin presents as a dry, nodular surface and with an aspect resembling leather, with deep wrinkles, accentuated furrows, bags and prominent parts. On the other hand, chronological aging (intrinsic) results in fine wrinkles, tuning and flabbiness of the skin (EMERIT, 1992; RANGARAJAN & ZATZ, 1999). Furthermore, in the photoaged skin, we have the presence of pre-malignant and malignant neoplasms (TAYLOR et al., 1990).

Those effects seem to be associated with the direct impact of photons in cellular DNA and with the direct impact of free radicals and oxygen reactive species generated from ultraviolet radiation absorption by photo-sensitive molecules (DELLA CARBONARE & PATHAK, 1992). The consequences of that impact are alteration of the gene expression pattern and the damage to cellular components (SCHARFFETER-KOCHANEK, 1997). An example is the metaloproteinases synthesis induction by the fibroblasts. These enzymes are responsible for tissue conjunctive components degradation, such as collagen, elastin, proteoglicans and fibronectin (SCHARFFETER-KOCHANEK, 1997; ONISHI et al, 2000).

On the other hand, the skin has a variety of antioxidants, such as enzymatic systems, low molecular weight polar and apolar antioxidants, capable of inhibiting the oxidative damage. The vitamin E is the most important exogenous lipophilic antioxidant found in the tissues. It acts together with antioxidant enzymes, such as gluthathione peroxidase, catalase and superoxide dismutase, as well as with smaller molecules as ascorbic acid, gluthathione and uric acid. The direct degradation of the α-tocopherol by UV radiation and the formation of its radicals can influence the other antioxidants of the system, as vitamin C. One of the first events in the cell exposed to ultraviolet radiation is the lipid peroxidation induction. "In vitro" studies, examining low density lipoproteins oxidation, demonstrated that the lipid peroxidation occurs when vitamin E is almost fully degraded. (ESTERBAUER *et al*., 1993). As aforementioned, the α-tocopherol indirect degradation occurs by the reaction with peroxyl radicals and the formation of oxidized products of α-tocopherol Such reactions occur predominantly in the cell lipid core.

There are at least three ways trough which the antioxidants concentration can be affected by ultraviolet radiation: (1) direct light absorption, (2) reaction with oxygen reactive species generated by the photosensitization reactions and (3) recovery mechanism in which an antioxidant is saved at the expenses of other (Lopez-Torres *et al*., 1998).

The chronic oxidative stress, as occurs in modern life due to the excessive exposure to the sun and to the atmospheric pollution increase, may justify a supplementation with antioxidants, in order to delay the cutaneous aging process (EMERIT, 1992).

In this context, the topical application of antioxidants has been considered a promising strategy in the prevention of this oxidative damage to the skin. Considerable pre-clinical (BISSET, 1990; LOPEZ-TORRES ET AL, 1997; FUCHS, 1998) and clinical (MAYER, 1993) data were obtained indicating the potential use of α-tocopherol in the photodamage prevention to the skin. Besides the α-tocopherol, other substances of vegetable origin, such as flavonoids and other fenolic compounds, have been proposed for topical application in order to prevent the photodamage to the skin (BONINA et al., 1996; SAIJA et al., 1998), offering advantages, as easy obtaining and smaller production cost.

Other antioxidants have been studied to be used in cosmetics in order to prevent the photodamage. Among them, vitamin C; nitrous acid compounds that control the peroxide formation; zinc and manganese ions that serve as nutrients for native dermis bacteria, accelerating the active substances secretion, such as the enzyme superoxide dismutase and before the α-tocopherol (FOX, 2000).

Among the employed strategies in the photodamage prevention to the skin, we can point out the use of protective lotions (BISSET et al., 1987), of tretinoin (FISCHER et al., 1998), of alpha-hydroxyacids (GILCHREST, 1996) and antioxidants (FOX, 2000). As aforementioned, the oxygen reactive species are partly responsible for the damage caused to the skin by the excessive exposure to ultraviolet radiation (FUCHS et al., 1989; SCHARFFETTER-KOSCHANEK, 1997). Therefore, a decrease in the oxygen reactive species load produced by the exposure to radiation UV represents a very promising strategy of protection against the photodamage.

Nowadays, attention is drawn to the antioxidants of vegetable origin. Antioxidants originating from natural products which offer new treatment possibilities for diseases mediated by oxidative stress. Some enzymes and high plants secondary compounds were capable of protecting tissues against oxidative stress through the free radicals and oxygen reactive species inhibition or capture. (LARSON, 1988). A "in vitro" study investigated the potential use of a rosemary extract (*Rosmarinus officinales*) in the photodamage control to the skin (FOX, 2000). The use of flavonoids (quercetin, hesperetin and apigenin) as photoprotecting agents was investigated. Due to the fact that they inhibit the lipid peroxidation of phosphatidilcholine liposomes exposed to UV and due to the fact that they present a good cutaneous permeation, it is suggested that the topical application of flavonoids might be a good option for the treatment of diseases caused or exacerbated by ultraviolet radiation in the skin (BONINA et al., 1996).

Brazil has a flora that is extremely rich in medicinal plants with great potential for supplying these antioxidant agents. Among these plants, the family Piperaceae, in particular the "pariparobas" are used thoroughly in popular medicine. The roots of *Pothomorphe umbellata* (L.) Miq. were included in Brazilian Pharmacopoeia's first edition (SILVA, 1926).

The *Pothomorphe umbellata* is a plant frequently found in the states of São Paulo, Minas Gerais, Espirito Santo and in the south of Bahia, and popularly known as "pariparoba". This is used in popular medicine for treatment of several illnesses, such as: hepatic insufficiency, indigestion, asthmatic bronchitis and externally in the treatment of burns and common wounds (MORAES, 1983). Brazilian Pharmaceutical Code's first edition depicts the flowing extract of pariparoba, the pariparoba syrup and the pariparoba depurative and ferruginous syrup (MORAES, 1983). The empiric observation of the physiologic action of that plant led to its employment internally and in small doses, as exciting of the stomach and liver functions, by increasing the appetite, activating the digestion such as an aromatic bitterness and promoting the bile drainage, as cholagogue that is. However, such plants are still being used and empirically as antispasmodic, which still has not been confirmed by clinical assays. Internally, these plants effects are recognized in the country, evidently, and have a great merit and acceptance, that comes from the aborigines (FREITAS, 1999; PECKOLT, 1941).

In relation to the antioxidant activity of the lyophilized extract of the *Pothomorphe umbellata* root, in vitro assays have evaluated it, using as model the self-oxidation of mouse brains, and such activity was partly attributed to the presence of 4-nerolidylcatechol (BARROS et al., 1996), a fenolic compound extracted from the vegetable roots (KIJJOA, 1980). In another in vitro assay, the *Pothomorphe umbellata* roots extract showed an antioxidant potential significantly larger than the one of this isolated compound, suggesting the presence of additional compounds with antioxidant activity (DESMARCHELIER et al., 1997). Based on these data, a study was accomplished seeking to evidence and evaluate the antioxidant activity of the *Pothomorphe umbellata* extract in the skin. The topical application of an lyophilized extract of the *Pothomorphe umbellata* roots on the skin of Hairless mice caused a reduction of 97% in the lipoperoxidation indicators as production of reactive substances to the acid tiobarbituric and chemiluminescence. This antioxidant activity was 2,5 times larger than the one of the α-tocopherol, a known antioxidant, applied to the same conditions (RÖPKE, 1999).

Although the antioxidant activity of *Pothomorphe umbellata* is known, there are no available information regarding the absorption of the active principle by the skin and about the most appropriate formulation to serve as vehicle for this drug. Furthermore, there are not specific studies about the performance of the active principle of this plant in the oxidative stress caused by the ultraviolet radiation.

### Objects, Advantages and Solutions

The present invention intends to demonstrate the antioxidant and inhibitory action of the lipoperoxidation in the skin presented by the pariparoba extract (*Pothomorphe umbellata*)*,* the use of a gel composition of the invention for the preparation of dermocosmetic or pharmaceutical compositions capable of treating and/or preventing the photodamage in the skin caused by the excessive exposure to the sun and to help in the treatment of photoaging and/or skin cancer, as an alternative to the substances of vegetable origin presented in the state of the art.

The employment of *P. umbellata* extract in the preparation of a medicament is quite interesting for its economic feasibility, due to the easy access to the raw material, as it is very common Brazilian plant with low production cost, and due the fact that the extract can be obtained in a simple way, not involving complex and costly techniques.

### Drawings

In the following, the invention will be described and for a better understanding, figures will be presented:
**Figure 1****:** Acid ascorbic concentration (µg/g of skin) in the skin of mice. The values are represented as percentages of the average acid ascorbic concentrations in the skin of control mice (174.61 ± 7.76 µg/g of skin); percentage of the irradiated control group (C + UV), 80.063 ± 14.57; of the gel irradiated control group (G + UV) 78.59 ± 19.04 and of the *P. umbellata* gel treated group (Pu + UV), 103.74 ± 22.04. The difference among the groups was not considered significant. The values were obtained from the average of the results of seven animals.
**Figure 2****:** α-tocopherol concentration (µg/g of skin) in the skin of the mice. The values are represented as percentages of the average α-tocopherol concentrations in the skin of mice of control group (2.70 ± 0,69 µg/g of skin). Percentage of the irradiated control group (C + UV), 35.05 ± 11.26; of the gel irradiated control group (G + UV) 42.90 ± 20.28 and of the *P. umbellata* gel treated group (Pu + UV), 106.77 ± 28.
   *p <0,001, considered very significant when compared to the control group. The values were obtained from the average of the results of seven animals.
**Figure 3****:** 4-nerolidylcatechol concentration in the skin of irradiated and not irradiated mice, treated with *P. umbellata* gel (Pu, 14.14 ± 3.09 µg/g of skin) and with radiation UVB (Pu + UV, 16.00 ± 2.38µg/g of skin). The data represent the average and the standard deviation of 6 animals.
**Figure 4****:** Hairless mouse skin exposed to ultraviolet radiation (degree 4).
**Figure 5****:** Hairless mouse skin exposed to ultraviolet radiation and treated with the gel containing *Pothomorphe umbellata* extract (degree 1).

### Description of the Invention

In the present invention, the pariparoba (*Pothomorphe umbellata*) extract is extracted from the root of this plant (commonly found in the south-eastern area of Brazil). It presents, among others, antioxidant activity, preventing the photodamage caused by the excessive exposure to the sun and to artificial tanning lamps. It also presents inhibitory action of the lipid peroxidation, which is one of the first events to occur in the cell exposed to ultraviolet radiation.

The skin is equipped with a variety of antioxidants as enzymatic systems, low molecular weight polar and apolar antioxidants, capable of inhibiting the oxidative damage. The α-tocopherol indirect degradation, that is an antioxidant, it occurs through the reaction with peroxyl radicals and formation of α-tocopherol oxidated products. These reactions occur predominantly in the cell lipidic medium. One of the performing forms of the *P. umbellata* extract is over those antioxidants in order to prevent the effects caused by the skin oxidative stress, that is, the 4-nerolidylcatechol found in the pariparoba extract acts on the α-tocopherol in order to avoid its degradation and consequently the one of other antioxidants of the enzymatic system.

The pariparoba (*P. umbellata*) antioxidant action, as mentioned above, is caused partly by the presence of the 4-nerolidylcatechol in the extract of that plant. However, as previously seen, studies (BARROS et al., 1996) showed that the extract, when being used, presented higher activity than the 4-nerolidylcatechol in an isolated way. Therefore, the proposed invention uses the extract obtained from the root of that plant for the obtaining of dermocosmetic or pharmaceutical compositions.

### Pharmaceutical Composition

Another aspect of the present invention refers to the use of a gel composition of the invention for the preparation of a dermocosmetic or pharmaceutical composition, which comprises as an active ingredient, the *P. umbellata* root extract. Composition shall be considered as the group formed by the active principle and the other ingredients (pharmaceutically acceptable excipient) that form the carrier, as well as any product that results, direct or indirectly, from the dissociation of one or more of the ingredients, or of other types of reactions or interactions of one or more of the ingredients.

The gel composition, exemplified in the form of a dermocosmetic, for use according to the proposed invention, can be prepared in accordance with prior art methods, for topical use. This example illustrates the chosen formulation, but it does not intend to limit the invention in any way. A proposed composition is comprised of:
a) carboxymethylcellulose 0.01 - 10.0%
b) propyleneglycol 0.001 - 50.0%
c) methylparaben 0.001 - 3.0%
d) *Pothomorphe umbellata* standardized extract, so that the formulation contains 0.005 to 20.0% of 4-nerolidylcatechol
e) distilled water q.s.p. 100.0%

For the preparation of an effective gel composition, it is necessary that the extract standardization in regard to the amount of 4-nerolidylcatechol present therein. This is made in a high efficiency chromatography device coupled to an electrochemistry detector or UV detector.

The preparing of the gel composition for use according to this invention can be made through any known methods in the pharmacy art and in combination with an pharmaceutical carrier, in accordance with the conventional techniques of pharmaceutical composition.

After standardizing the extract in regard to its concentration of 4-nerolidylcatechol, then, it is incorporated in the cosmetic and/or pharmaceutical bases, so that to reach the desired active principle concentration.

In all possibilities, they must be sterile and stables in the manipulation and storage conditions and preserved against polluting action of microorganisms, as bacteria and fungi.

The carrier, in addition to the mentioned ones in the proposed composition, can be a solvent or a dispersion medium containing, for example: water, ethanol, polyol (for example, glycerol, propylene glycol and liquid polyethylene glycol), appropriate mixtures and vegetable oils. In addition to aforementioned the ingredients, the described pharmaceutical formulations can include, in an appropriate way, one or more carrier ingredients, such as diluents, buffers, ligants, surface active agents, thickeners, preservatives (including anti-oxidizers) and similar and inclusion of other substances.

### Doses

The effective dosage of the used ingredient can vary depending on the way of presentation of the dermocosmetic and/or pharmaceutical composition, the condition to be treated and the severity of the condition to be treated, so that the amount of the *P. umbellata* extract topically administered is effective. In a general way, it can be said that the formulation will be effective with the presence of at least 0.1% of 4-nerolidylcatechol.

The capacity of the pariparoba extract and accordingly of the 4-nerolidylcatechol of acting in the treatment and/or prevention to the photodamage to the skin, cutaneous aging and skin cancer can be illustrated together with the following non-limiting examples and figures.

**Example 1** - Effect of the topical application of *Pothomorphe umbellata* root extract on the low molecular weight antioxidants, in the oxidative stress of the skin.

Hairless male mice were used (HRS/J), with approximately 10 weeks of age. They were divided into four groups, each one having 7 animals. The first group received topical treatment with gel base without the presence of *P. umbellata* and the second group with *P. umbellata* gel with concentration of 0.1% of 4-nerolidylcatechol. The third group did not receive any treatment. After two hours, the animals were irradiated by a Philips lamp TL 12/40W for 30 min with only one dose of UVB radiation (290-320 nm), 26 cm distant. This irradiation corresponds to 22.95 x 10⁻² J/cm², twice the minimum dose to cause erythema in these animals. The radiation was measured with a UVB sensor. Still, a fourth group of mice did not receive treatment, nor it was irradiated.

After the irradiation, the animals were sacrificed by cervical displacement. The 4-nerolidylcatechol absorbed by the skin and the α-tocoferol of the sacrificed mice tissues were extracted based on the method proposed by Burton (BURTON AND INGOLD, 1985) and, then, it was made the quantitative analysis of these components through the chromatographic method. Was also quantitatively analyzed the ascorbic acid, through the methodology proposed by Wayner & Burton (WAYNER & BURTON, 1989).

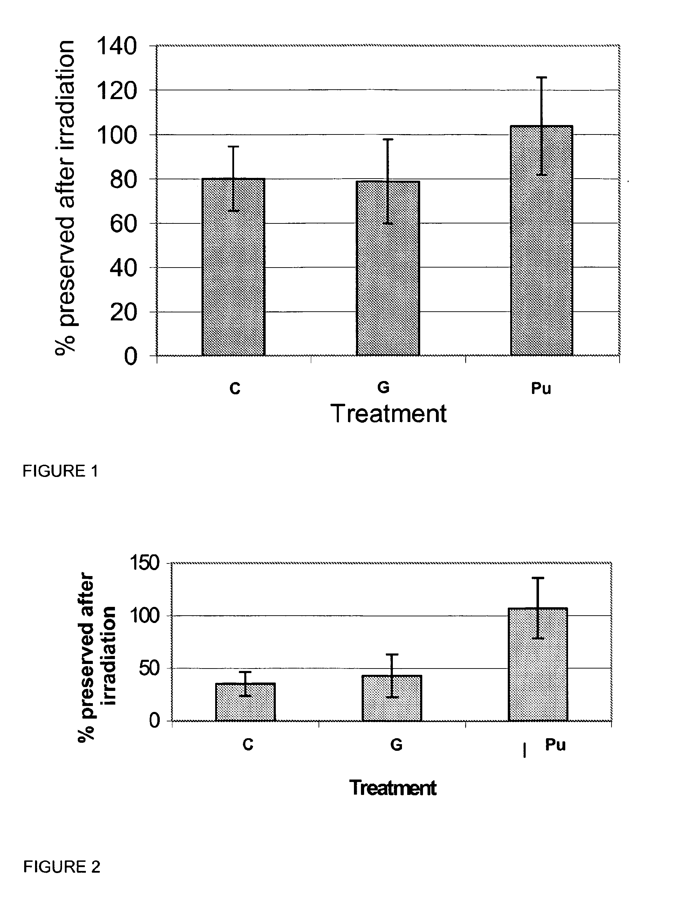

The obtained results are shown in the figures 1 to 3. As shown in the figure 1, no significant alteration was noticed in the levels of ascorbic acid in the skin of irradiated mice, possibly due to the used dose and to the UV λ used. In the figure 2, it is possible to observe that the topical treatment with *P. umbellata* preserved the levels of α-tocopherol in the skin of irradiated mice, protecting itself against degradation for the UV radiation. After the irradiation, there was a significant decrease (p <0.01) in the levels of α-tocopherol in the skin of the mice from the control group irradiated, while in the treated group with *P. umbellata* gel, the α-tocopherol was preserved (p> 0.05).

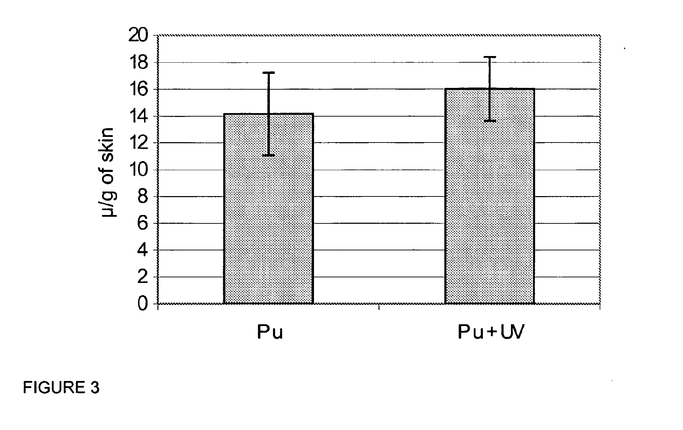

No Difference was observed among the 4-nerolidylcatechol concentration in the skin of irradiated and not irradiated mice, as shown in figure 3. The similar chemical nature of the α-tocopherol and of the 4-nerolidylcatechol can increase its interaction, what explains the preservation of the levels of α-tocopherol in the mice treated with the *P. umbellata* extract. In the skin of the animals irradiated with UVB (0.3 J/cm²), there was a reduction of approximately 60% in the α-tocopherol concentration.

**Example 2** - Effect of the topical application of formulation on the basis of the *Pothomorphe umbellata* root extract in the prevention of photoaging caused by the chronic exposure to ultraviolet radiation.

It was used in this example a gel formulation containing the *Pothomorphe umbellata* extract which obtained in the previous tests the best liberation of active principle in the skin. A UVB Philips lamp TL-12 rs 40W was used for irradiation of Hairless mice. The animals were irradiated four times a week, for 10 min, during a period of 22 weeks. The used dose was of 76.5 mJ/cm² (approximately 0.7 times the necessary minimum dose to cause erythema in the animals). The dose was measured in a radiometer, with a UVB SED 240 sensor. Two hours before the irradiation, the mice were treated with gel containing the *Pothomorphe umbellata* extract, in the concentration of 0.1% of 4-nerolidylcatechol. At the same time, mice without treatment and treated with gel without active principle as control groups were irradiated. A fourth control group was not treated nor irradiated.

The formation of wrinkles was classified according to the proposed score in the experimental model of photoaging presented by Bisset (BISSET et al., 1987).

**Table 1: Score for evaluation of the wrinkles formation degree in the skin of Hairless mice.**

| **DEGREE** | **Skin Description** |
|---|---|
| 0 | Numerous fine grooves covering the back and the sides of the body, that appear and disappear with the movement. |
| 1 | All the fine grooves in the back and along the spine disappear. Few superficial and rough wrinkles perpendicular to the head-tail direction, that appear and disappear with the movement. |
| 2 | Rough and permanent wrinkles along the body. |
| 3 | Deep and permanent wrinkles. Leathery skin with no elasticity. |
| 4 | Skin with coriaceous aspect having lesions of dark coloration in the back. |

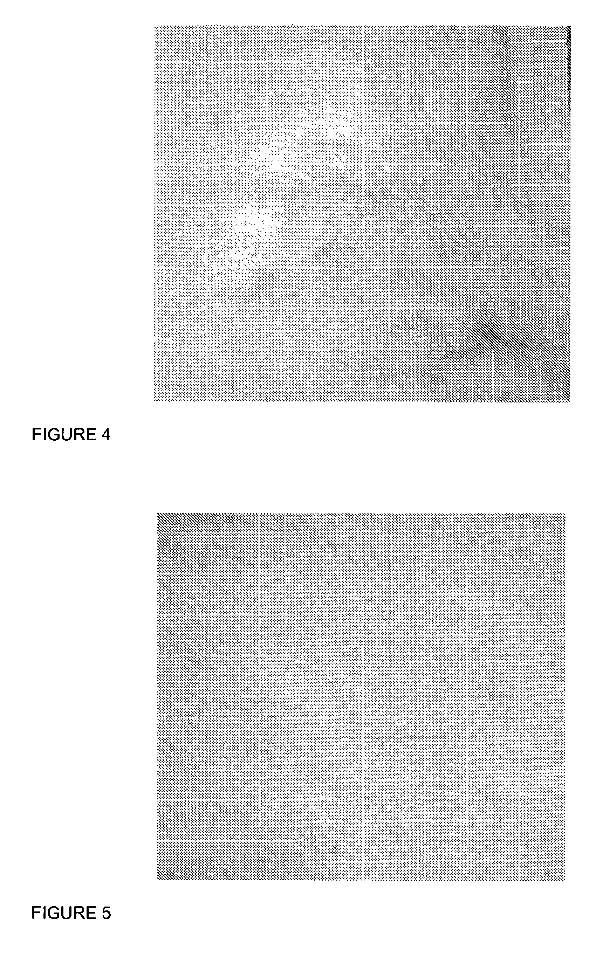

The mice treated with *P. umbellata* gel (figure 5), visibly did not suffer the consequences of the chronic exposure to the ultraviolet radiation, as much as those which were irradiated without being treated with the *P. umbellata* gel (figure 4). This study proved the effectiveness of the formulation in the prevention of photoaging caused by the chronic exposure to ultraviolet radiation. With regard to the animals of other groups, alterations caused by photoaging were noticed, as such deep and permanent wrinkles, leatheriness and even lesions were visible. It is being demonstrated through to present invention an easy form to obtain *Pothomorphe umbellata* extract and its effectiveness as an antioxidant and inhibitor agent of the lipid peroxidation in the skin, it makes of formulations containing this extract an important alternative for treatment and/or prevention to the photodamage in the skin, cutaneous photoaging and skin cancer, which are one of the health agents' most serious concerns, due to the increase of the exposure to ultraviolet radiation caused by the decrease of the ozone layer of Earth.

## Claims

1. Gel composition for use in the treatment and/or prevention of photodamage to skin, cutaneous ageing and/or skin cancer, on the basis of *Pothomorphe umbellata* extract comprising a standardized extract of *Pothomorphe umbellata* which contains a range from 0.005 to 20.0% of 4-nerolidylcatechol in the composition.

2. Gel composition for use according to claim 1 wherein said composition is presented for topical use.

3. Gel composition for use in the treatment and/or prevention of photodamage to skin, cutaneous ageing and/or skin cancer comprising:
a) carboxymethylcellulose from 0.01 to10.0%,
b) propyleneglycol from 0.001 to 50.0%,
c) methylparaben from 0.001 to 3.0%,
d) Pothomorphe umbellata standardized extract, so that the formulation contains 0.005 to 20.0% of 4- nerolidylcatechol.

4. Gel composition for use according to claim 3 wherein said composition is presented for topical use.

5. Use of a gel composition according to any one of the claims 1 to 4 for the preparation of a dermocosmetic or a pharmaceutical composition for the treatment and /or prevention of photodamage in skin, cutaneous aging and/or skin cancer.

6. Use of a gel composition according to any one of the claims 1 to 4 for the preparation of a dermocosmetic or a pharmaceutical composition for the treatment and /or prevention of skin damage caused by the excessive exposure to ultraviolet rays of sun and/or to artificial tanning lamps and alterations caused by chronological aging.

## Patentansprüche

1. Gelzusammensetzung zum Einsatz bei der Behandlung und/oder Vorbeugung von Lichtschäden an der Haut, Hautalterung und/oder Hautkrebs, auf der Basis eines *Pothomorphe-umbellata*-Extrakts mit einem standardisierten Extrakt von *Pothomorphe umbellata,* der 0.005 bis 20.0% 4-nerolidylcatechol in der Zusammensetzung enthält.

2. Gelzusammensetzung zum Einsatz nach Anspruch 1, wobei besagte Zusammensetzung zur äußeren Anwendung dient.

3. Gelzusammensetzung zum Einsatz bei der Behandlung und/oder Vorbeugung von Lichtschäden an der Haut, Hautalterung und/oder Hautkrebs mit:
a) Karboximethylzellulose 0.01 bis 10.0%,
b) Propylenglycol 0.001 bis 50.0%,
c) Methylparaben 0.001 bis 3.0%,
d) standardisiertem *Pothomorphe-umbellata*-Extrakt, so dass die Formulierung 0.005 bis 20.0% 4- Nerolidylcatechol enthält.

4. Gelzusammensetzung zum Einsatz nach Anspruch 3, wobei besagte Zusammensetzung zur äußeren Anwendung dient.

5. Verwendung einer Gelzusammensetzung nach einem beliebigen der Patentansprüche 1 bis 4 zur Herstellung einer dermokosmetischen oder einer pharmazeutischen Zusammensetzung zur Behandlung und/oder Vorbeugung von Lichtschäden an der Haut, Hautalterung und/oder Hautkrebs.

6. Verwendung einer Gelzusammensetzung nach einem beliebigen der Patentansprüche 1 bis 4 zur Herstellung einer dermokosmetischen oder einer pharmazeutischen Zusammensetzung zur Behandlung und /oder Vorbeugung von Hautschäden aufgrund übermäßiger Aussetzung zu ultravioletten Sonnenstrahlen und/oder künstlichen Bräunungslampen sowie von Veränderungen aufgrund des Älterwerdens.

## Revendications

1. Composition de gel pour l'utilisation dans le traitement et/ou la prévention de la photosensibilisation, du vieillissement de la peau et/ou du cancer de la peau, à base d'extrait de *Pothomorphe umbellata* comprenant un extrait standardisé de *Pothomorphe umbellata* qui contient de 0.005 à 20.0% de 4-nérolidylcatéchol dans la composition.

2. Composition de gel pour l'utilisation selon la revendication 1 dans laquelle ladite composition est destinée à l'utilisation topique.

3. Composition de gel pour l'utilisation dans le traitement et/ou la prévention de la photosensibilisation, du vieillissement de la peau et/ou du cancer de la peau comprenant :
a) de 0.01 à 10.0% de carboxyméthylcellulose,
b) de 0.001 à 50.0% de propylène glycol,
c) de 0.001 à 3.0% de méthylparaben,
d) un extrait standardisé de *Pothomorphe umbellata,* de sorte que la formulation contient de 0.005 à 20.0% de 4-nérolidylcatéchol.

4. Composition de gel pour l'utilisation selon la revendication 3, où ladite composition est destinée à l'utilisation topique.

5. Utilisation d'une composition de gel selon l'une quelconque des revendications 1 à 4 pour la préparation d'une composition dermo-cosmétique ou pharmaceutique pour le traitement et/ou la prévention de la photosensibilisation de la peau, du vieillissement de la peau et/ou du cancer de la peau.

6. Utilisation d'une composition de gel selon l'une quelconque des revendications 1 à 4 pour la préparation d'une composition dermo-cosmétique ou pharmaceutique pour le traitement et/ou la prévention du vieillissement de la peau provoqué par l'exposition excessive aux rayons ultraviolets du soleil et/ou aux lampes à bronzer et des altérations causées par le vieillissement chronologique.
